# EUROPEAN PATENT APPLICATION

(11) **EP 2 395 338 A1**
(43) Date of publication of application: **14.12.2011**
(21) Application number: 10425193.9
(22) Date of filing: 10.06.2010
(51) Int. Cl.: G01N 1/04, A61B 10/02, A61B 10/00

(54) **Device for obtaining DNA samples**

(71) Applicant: Gentras S.r.l., 59100 Prato (IT)
(72) Inventor: Quattrone, Alessandro, 59100 Prato (IT); Quattrone, SIlvia, 59100 Prato (IT); Cesati, Valentina, 59100 Prato (IT); Frosini, Gianluca, 59100 Prato (IT)
(74) Representative: Leihkauf, Steffen Falk

(57) **Abstract**

The object of the present invention is a device for collecting skin cells (7,27,407), adapted for the transport, preservation and subsequent extraction of the DNA and/or RNA contained in the drawn sample. The device has characteristics which make it economical to produce and simple to use, and it is structured in such a manner that the risk of cross contamination is strongly limited.

## Description

The object of the present invention is a non-invasive device for the collection, transport and preservation of skin cell samples. The device that is described herein has characteristics which make it cheap to produce and simple to use, and is structured in such a manner as to strongly limit the risk of cross contamination. The device is therefore usable in any situation in which it is necessary to obtain, in a non-invasive manner, a biological sample from which the DNA of a given subject is then extracted.

### State of the art

Genetic patrimony is preserved in DNA, present in every cell of the organism. The DNA of every individual encloses information sufficient for his unequivocal identification. Such information is obtainable by analyzing the DNA in question by means of techniques known in the state of the art (Sambrook J and Russell DW. Molecular cloning: a laboratory manual. Cold Spring Harbor Press. 2001) which allow highlighting its homologies and peculiarities with respect to other known DNA. For this reason, in legal venues, the DNA test aimed to the identification of people is now in force. The observation of increasingly numerous correlations between genes and disease predisposition, between genes and drug treatment response, between genes and harmful substance effect and finally between genes and diet components has added further interest in the analysis of a subject's DNA. From this brief account, it is clear that the knowledge of characteristics of the individual's DNA has enormous potential, its applications range from diagnostic and prognostic tests for genetic diseases and complex diseases, to genomic drug tests, toxicogenomics and nutrigenomics, from the unambiguous identification of individuals to paternity tests. A technology that powerful must be supported by a rigorous, efficient and practical collection of samples from which the DNA itself is obtained, which assures its origin, a purity and quality suitable for carrying out the test, and which excludes the risk of cross contamination. Conventionally, when the reference analysis was the RFLP (Restriction Fragment Length Polymorphism), the DNA of an individual was obtained from the blood, with a series of non-negligible ethical, sanitary and compliance implications of the subject. Then, DNA came to be extracted from saliva, by means of the use of buccal pads. With the refinement of the DNA extraction and analysis technologies, the possibility of obtaining quality DNA samples in sufficient quantities for the required purposes, also from skin cells, led to the development of several suitable systems. In such a manner, the disadvantages of working with liquid samples, subject to bacterial contamination and thus to degradation, were thus overcome.

W00017396 describes a rectangular adhesive surface for collecting skin cells that is sufficiently large for comprising the entire palm of the hand and/or the fingertips.

US20090126625 describes an adhesive surface covered with graphite that is sufficiently large for comprising the fingertips, so as to obtain with the sampling both the skin cells from which the DNA is then extracted and the fingerprints of the same subject.

W02009/123373 describes a skin card constituted by a support and an adhesive portion for the collection of DNA and RNA samples from the skin.

None of the above mentioned documents describes devices provided with structural expedients such to facilitate the collection, transport, preservation and elution of the sample and capable of preventing the risk of cross contamination, requirements that are strongly felt with the diffusion of the applications based on DNA analysis. As an example, DNA analysis will be a priority in order to expose a subject to a personalized drug therapy. For this purpose, it will be necessary to collect the DNA of the patient in order to then be able to analyze it at specialized centers. So that this process can occur in a routine manner, the need is strongly felt to provide a device that allows, in a simple manner and with limited costs, the collection, transport and preservation of the biological sample and finally the extraction of the DNA from the same, in a quantity and with a level of integrity suitable for allowing the analysis.

The object of the present invention is to provide a non-invasive system for obtaining DNA samples from subjects, characterized by simplicity of use, containment of achievement and use costs, and which is structured in a manner such that it strongly limits the risk of cross contamination.

The right to privacy, the traceability of the sample and the analysis of the same in separate places and/or at separate times are also assisted by the device claimed herein.

### Description of the invention

The object of the present invention is a device for collecting skin cells, adapted for transport and preservation and for the subsequent extraction of the DNA contained in the drawn sample.

One of the particular characteristics of the device is the elimination of the risk of cross contamination, both in drawing step and in transport and preservation step. Further particular features and characteristics emerge from the description and from the claims which follow and which form an integral part of the present description.

### Description of the figures

Figure **1****:** Perspective view of the external face **(A)** and the internal face **(B)** of one embodiment.
Figure **2****:** Perspective view of the internal face of a second embodiment.
Figure **3****:** Perspective view of the internal face of a third embodiment.
Figure **4****:** View of a fourth embodiment. **(A)** perspective view of the spread-out external face. **(B)** perspective view of the spread-out internal face.
Figure **5: (A)** perspective view of the device in the fourth embodiment ready for use. (**B**) , (**C**) perspective view of the device in closure step after the collection of the sample. **(D)** perspective view of the closed device containing the sample.

### Detailed description of the invention:

In the embodiment 1, the device is a support sheet 7 having a line of demarcation along the symmetry axis for dividing the support itself in two symmetrical halves. The flat sheet 7 comprises an internal face 107 and an external face 207. The internal face 107 comprises an adhesive portion 7a and an anti-adhesive portion 7b. The device comprises a protective film 3 to cover the adhesive portion 7a, a protective membrane 8 to cover the anti-adhesive portion 7b and a protective membrane 9 to cover the external face 207. The detachment of the protective membranes 8, 9 leaves the sheet clean.

In one embodiment, such sheet will have an area comprised between 10 and 120 cm², more preferably comprised between 20 and 60 cm², more preferably it is a rectangular of about 50 cm² surface area. The adhesive portion 7a comprises an adhesive film 2.

The anti-adhesive portion 7b will be treated in such a manner to result anti-adhesive.

The sheet 7 is constituted by any solid material which can be prepared in sheet form and covered with an adhesive. As an example, can be used paper supports, synthetic resins such as cellulose, polypropylene, polyethylene and PVC, fibers, such as those used in adhesive plastic or metals, such as aluminum. The adhesive film 2 can be of any type. In particular, adhesives can be used which are not harmful when applied on the human body. As an example, 3M adhesives can be used of 1500, 1522 or 9874 type, or Scotch™, Wave Solder Tape 5414.

The protective film 3 has a length or width extension greater than the adhesive portion 7a, so as to present a strip for the peeling.

The sheet 7, once the protective film 3 has been removed, the sample on the adhesive portion 7a collected and the protection membrane 8 present on the anti-adhesive portion 7b removed, is folded along the line of symmetry 5, such that the adhesive portion 7a containing the drawn sample is situated in contact with the anti-adhesive portion 7b and thus remains protected in the transport and storage steps.

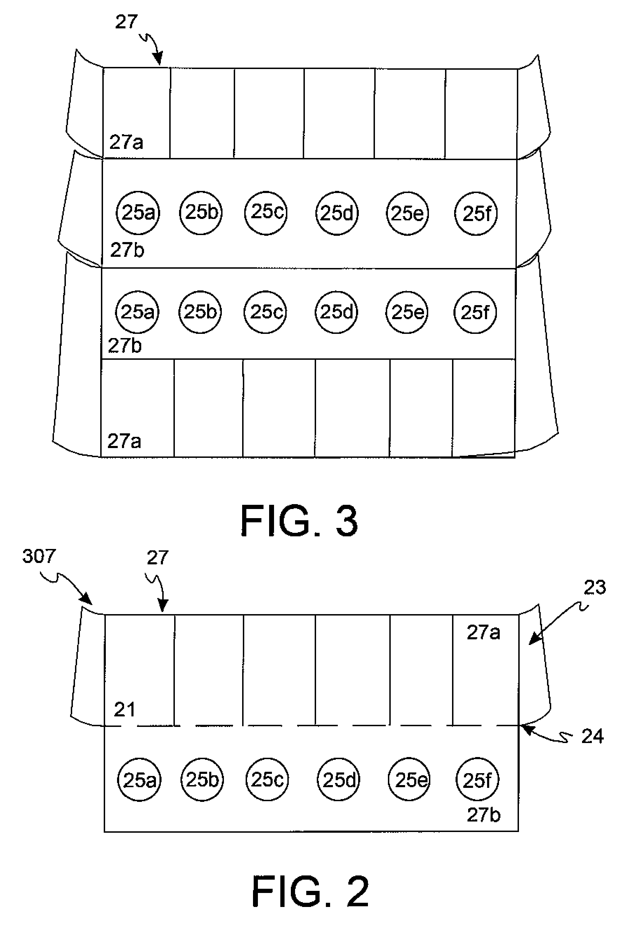

A second embodiment is described in Figure 2. Also in this embodiment, there is a support sheet 27 provided with a symmetry axis 24. The internal face 307 of said sheet comprises an adhesive portion 27a and a non-adhesive portion 27b. Here too, said adhesive portion 27a is covered by an adhesive film 21. The protective film 23 covering the adhesive portion 27a has a length or width extension greater than the adhesive portion, so as to present a strip for the peeling. Here too, a protective membrane is present for covering the external face (not shown). The materials which constitute the sheet, the adhesive film, the protective film and the protective membrane will be the same described for the previous embodiment.

This embodiment is characterized by low relief zones 25a, 25b, 25c, 25d, 25e, 25f placed on the non-adhesive portion 27b. Such low relief zones ensure, once the support is folded along the line of symmetry 24, the formation of an air chamber to delimit portions of the adhesive surface 21 containing the collected sample. Such low relief zones are preferably made of PVC. They constitute cavities that comprise a flat portion and an edge raised over the entire perimeter of the same. Such raised edge will have a height comprised between 0.1 and 5 mm. Alternatively, said low relief zones form concave cavities, with depth comprised between 0.1 and 5 mm. Such low relief zones could have circular form as indicated in the figure or any other suitable form, e.g. rectangular or square form.

Alternatively, the embodiment depicted in Figure 3 provides that the sheet 27 comprises a double series of low relief zones 25a, 25b, 25c, 25d, 25e, 25f to which two adhesive portions 27a correspond. In such device, the two adhesive zones 27a are opposite each other and externally situated with respect to the two non-adhesive portions 27b comprising the low relief zones, on which they will be folded. In this manner, a single device is available that offers a larger adhesive portion and hence the possibility of obtaining greater sample amounts.

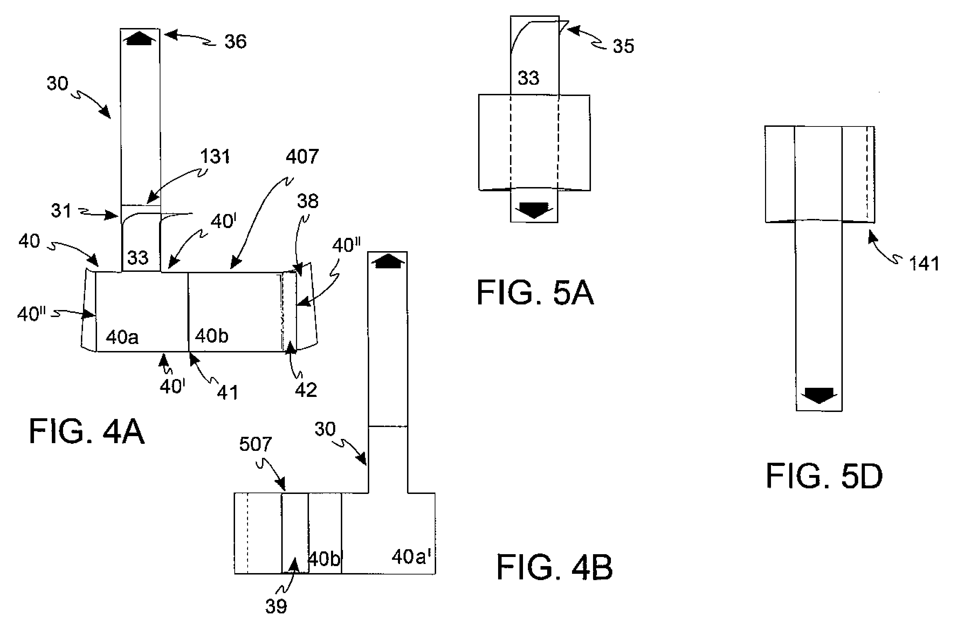

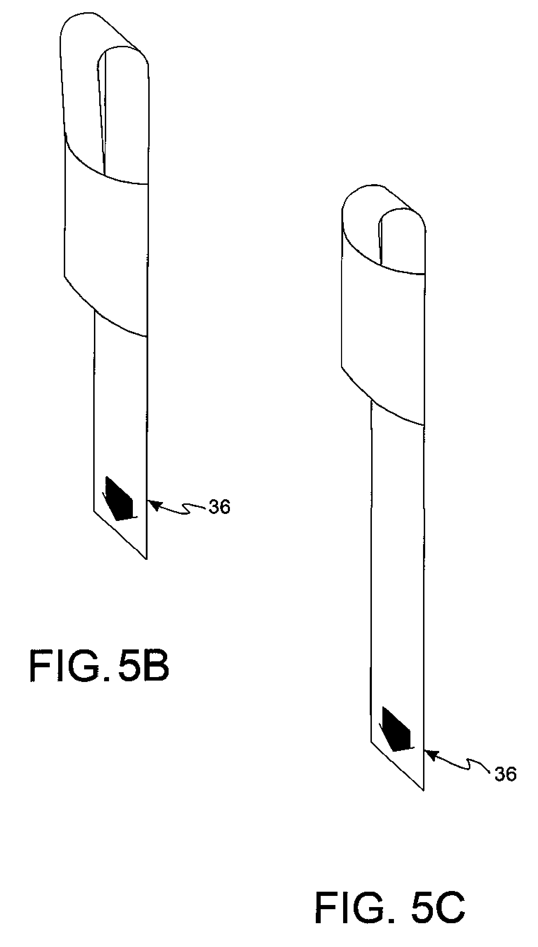

In a further embodiment, described in Figure 4A and 4B and 5A, 5B, 5C, it is provided that the collection surface, after having drawn the sample, can be housed in a suitable protective housing with a simple sliding movement. In particular, in the perspective view of Figure 4A and 4B, the external face 407 and the internal face 507 are depicted of the sheet 30 that constitutes the device of the invention. The device is shown in spread-out form. The external face 407 comprises a protective housing portion 40, which when folded will constitute the external handling side of the protective housing; and a tongue portion 31 that is extended from an edge portion of the protective housing portion 40.

The protective housing portion 40 is extended along a main axis, so as to define two opposite greater edges 40' and two opposite lesser edges 40".

The protective housing portion 40 is divided by a line of demarcation 41 perpendicular to said greater edges 40' which divides the protective housing portion 40 into two non-symmetrical portions, a smaller portion 40a and a greater portion 40b. The greater portion 40b comprises an end portion 42, in turn separated by a line of demarcation, which will serve as a closure of said protective housing.

The tongue portion 31, made integrally with the protective housing portion 40, is extended from a greater edge 40' of the smaller portion 40a of the protective housing portion 40, perpendicular to the main axis of the protective housing portion 40. In a different embodiment, the tongue portion 31 is extended from the greater portion 40b.

The tongue portion 31 comprises an adhesive portion 33. The adhesive portion 33, adjacent to the edge of the protective housing portion 40 from which it extends, is covered by an adhesive film. The distal end of the tongue portion 31 constitutes gripping means 36. The gripping means 36 and the adhesive portion 33 are delimited by a folding line 131.

In figure 4B, the internal face 507 of said sheet 30 is depicted. Such internal face 507 comprises an anti-adhesive portion 39 that, when the device is folded as in figure 5D, will be found at the adhesive portion 33 of the tongue portion 31. Consequently, such anti-adhesive portion is arranged in a substantially median position of the greater portion 40b of the protective housing portion 40.

In assembly step, the protective housing portion 40 is folded to form the protective housing and the tongue portion 31 is inserted in the protective housing itself as illustrated in figure 5A, so as to make the distal end 36 of the tongue portion 31 come out from the edge of the protective housing opposite the edge from which the tongue portion 31 is extended. In such configuration, the adhesive portion 33 extends on the top from the edge of the protective housing itself.

The adhesive portion 33 is protected by a protective film 35 to be removed at the time of the sample collection. The protective film 35 has a length or width extension greater than the adhesive portion 33, so as to present a strip for the peeling.

After collection, the adhesive portion 33 containing the sample will be made to slide inside the protective housing by applying a traction of the tongue portion 31, grasping the gripping means 36 as depicted in figures 5B and 5C. In such a manner, the adhesive portion 33 containing the drawn sample will be found inside the protective housing, in contact with the anti-adhesive portion 39 as depicted in figure 5D. As in the previous embodiments, also this embodiment provides for covering the external face 407 with a protective membrane 38. The protective housing is easily openable in order to allow the retrieval of the samples. The opening of the protective housing can be achieved through a weakness line 141 which for example is arranged, as in figure 5D, along the short side of the protective housing itself in order to allow a window opening, or through other suitable systems. The adhesive portion 33, the protective housing portion 40 and the tongue portion 31, like the protective film 35 and the protective membrane 38, are constituted by the same materials described for the preceding embodiments.

One alternative applicable to all the described embodiments provides for the possibility of detaching the adhesive film 2, 21, 33 from the adhesive portion 7a, 27a, 33. In this embodiment, a bi-adhesive film will be used, selected in such a manner that it has greater adhesive strength on the side in contact with the support than the adhesive strength present on the side to be used for the sample collection. The materials composing the adhesive and the support were selected so as to allow the operator to detach the adhesive from the support during the analysis step but to prevent the adhesive from detaching from the support during the sample collection step. In this embodiment, the adhesive film 2, 21, 33 can in turn be constituted by one or more portions which can be separated, as shown for example for the embodiment of figure 2. In particular, it can comprise from 1 to 10 adhesive strips, preferably 6 adhesive strips arranged in a contiguous manner on the support so as to form the total adhesive surface.

In particular, considering the embodiment 20, one expedient provided is that of arranging an adhesive film 21 constituted by the same number of portions as there are low relief zones 25a, 25b, 25c, 25d, 25e, 25f and having shape and size such to be contained inside the low relief zones themselves; adhesive strips and low relief zones will be arranged in mirror-like manner with respect to the symmetry axis 24.

Optionally, in the embodiments described, detachable self-adhesive labels will be present containing an ID code of the sample, constituted by an alphanumeric code or a bar code. Said labels can be present on the sheet 7, 27, on the protective housing portion 40 or on the tongue portion 31, or on a portion added for such purpose and positioned in a manner such that it does not interfere with the use of the device itself.

The devices depicted in the figures have rectangular shape; nevertheless, further embodiments are possible which provide for the use of circular or square surfaces, or of any polygonal shape having at least one symmetry axis.

The device, in any one of its described embodiments, can be previously treated with DiEthylPyroCarbonate (DEPC) in order to prevent the degradation of DNA and RNA by deoxyribonuclease and ribonuclease, respectively.

In addition, the device can be previously sterilized, preferably in an autoclave.

### Use and advantages

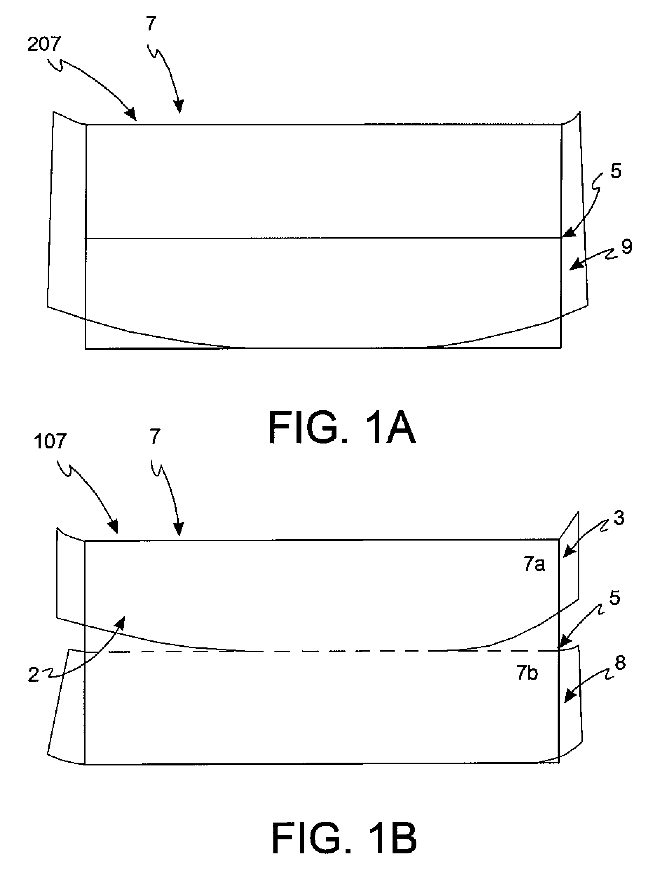

Using any one of the described embodiments, the collection of the sample will be carried out by removing the protective film 4, 23, 35 and setting with a slight pressure the adhesive portion 2, 21, 33 thus uncovered on the skin of the subject. The fact that the protective film is extended for a greater portion than the adhesive portion prevents cross contamination and facilitates the detachment of the protective film 3, 23, 35 by the subject himself or by the charged operator. The adhesive portion can be detached and reattached several times to the skin, so as to maximize the number of collected cells. The skin will have to be cleaned and dried, so as to avoid dust or moisture possibly present from interfering with the adhesive capacity. The adhesive portion of the device will then be folded on the support, in the embodiments of Figures 1, 2, and 3, or drawn into the protective housing, in the embodiment of Figure 4, such that the adhesive zone containing the sample is protected from external agents. In such a manner, the support itself, in its clean, anti-adhesive portion - clean since protected up to that moment by the protective film or because it is inside the protective housing - will act as a protection for the adhesive area now containing the sample. If present, the low reliefs in PVC will constitute an air chamber capable of protecting the sample from degradation due to sudden temperature changes or excessive humidity.

Optionally, the device can be part of a kit which also comprises a bag, preferably impermeable and sealable, where the device itself is inserted and housed. The sample collected by means of the device and thus housed can be easily transported or archived.

At the time of analysis, the operator will take the device from the bag, where it is present, eliminate the protective membrane 7, 26, 38 and draw the adhesive portion 2, 21, 33 containing the sample.

Where provided, the operator will detach only one or more of the adhesive portions from the support to use them for the extraction, preserving the remaining portions for subsequent tests or for delivery to third parties.

In particular, in the embodiment that provides for the use of low relief zones, in order to extract the DNA and/or RNA only from that sample portion which was preserved inside the air chamber formed due to the low reliefs, the adhesive film portions will be taken that are contained in the same. This embodiment is particularly recommended if the collected biological samples must be exposed to extreme environmental conditions for long time periods.

Where present, the ID labels will accompany the samples through the different working steps.

The presence of a label-code integral with the support that contains the sample favors the traceability of the sample itself in the multiple steps from the drawing to the extraction and the data analysis. Indeed, it should be considered that the collection and analysis of the data occur in different places, are managed by different people, and time periods can elapse between the two activities that range from a few hours to months or years. In addition, the association of a sample with an ID code eliminates the need to use the first name and last name of the subject under examination, ensuring the necessary privacy and maintaining the traceability. A suitable register with controlled access will ensure the ID management.

The DNA and/or the RNA will be extracted from the skin cells contained on the adhesive portion of the device by following known methods (Sambrook J and Russell DW. Molecular cloning: a laboratory manual. Cold Spring Harbor Press. 2001), or variants thereof. Given the need to immerse the device portion containing the sample in the extraction solution, it is advisable that such portion is as small and thin as possible, so as to be able to use a volume of extraction solution that is equally reduced. This allows simplifying the extraction procedure, limiting its costs, and - of no small importance - increasing the concentration of the extracted DNA. Since the use of very thin adhesive supports would be inconvenient in the sample collection step, it is clear that being able to arrange systems where the adhesive film 2, 21, 33 containing the sample can be detached from the adhesive portion offers considerable advantages.

The materials selected for the preparation of the adhesive and the support, where the adhesive is integral to the support itself, are such that they do not interfere with the process of extraction of the DNA and/or of the RNA and with the subsequent analysis.

From the skin samples collected with the device described herein, it is possible to extract DNA and/or RNA with known methods, obtaining a DNA and a RNA of a quality such that they can be used in any one of the currently available methods for DNA and RNA analysis, such as sequencing with Sanger method or with Next Generation Sequencing method, hybridization with probe systems of various types, including microarrays, assays of single nucleotide polymorphism (SNP) executed with various methods, mutation assays, methylation assays, possibly following amplification by means of Polymerase Chain Reaction (PCR) or Reverse Transcription (RT-PCR).

In order to verify the stability of samples collected and preserved with the device described herein, accelerated stability tests were conducted which showed that after a time equal to at least 25 years, it is possible to proceed with the extraction, obtaining DNA of a quality such to allow its amplification via PCR and subsequent analysis.

## Claims

1. Device for the collection, transport and preservation of skin cells which comprises a sheet (7, 27, 30) having an adhesive portion (7a, 27a, 33) for the collection of the sample and an anti-adhesive portion (7b, 39) or non-adhesive portion (27b), **characterized in that** said anti-adhesive or non-adhesive portion is intended to contact or to be facing said adhesive portion after the drawing of said sample.

2. Device according to claim 1, wherein DNA and/or RNA is extracted from said skin cells.

3. Device according to any one of the claims 1 or 2, wherein said adhesive portion comprises an adhesive film that is not harmful when applied onto the human body, preferably a 3M adhesive film of type 1500, 1522 or 9874, or Scotch™, Wave Solder Tape 5414.

4. Device according to any one of the claims 1 to 3, wherein said sheet is made of paper, of synthetic resins such as cellulose, polypropylene, polyethylene and PVC, of fibers such as those used in adhesive plastics, or of metals such as aluminum.

5. Device according to any one of the claims 1 to 4, which comprises a protective film (3, 23, 35) for covering the adhesive portion (7a, 27a, 33).

6. Device according to any one of the claims 1 to 5, wherein said protective film (3, 23, 35) has a length or width extension greater than the adhesive portion (7a, 27a, 33) so as to present a strip for the peeling.

7. Device according to any one of the claims 1 to 6, which comprises a protective membrane (8) for covering the anti-adhesive portion (7b) and a protective membrane (9, 38) for covering the external face (207, 407) of said sheet (7, 27, 30), wherein the detachment of said membrane leaves the sheet clean.

8. Device according to any one of the claims 1 to 7, wherein said sheet has a line of demarcation along the symmetry axis for dividing the support itself into two symmetrical halves and it comprises, on the internal face, an adhesive portion (7a) covered by an adhesive film (2) and an anti-adhesive portion (7b) and said sheet has an area comprised between 10 and 120 cm², more preferably comprised between 20 and 60 cm², more preferably it is a rectangle with about 50 cm² surface area.

9. Device according to any one of the claims 1 to 8, wherein said sheet (27) is provided with a symmetry axis (24) and comprises, on the internal face (307), an adhesive portion (27a) covered by an adhesive film (21) and a non-adhesive portion (27b).

10. Device according to any one of the claims 1 to 9, wherein on said non-adhesive portion (27b), low relief zones (25a, 25b, 25c, 25d, 25e, 25f) are placed.

11. Device according to any one of the claims 1 to 10, wherein said low relief zones are preferably made of PVC and come to constitute cavities which comprise a flat portion and an edge raised over the entire perimeter of the same.

12. Device according to any one of the claims 1 to 11, wherein said raised edge has a height comprised between 0.1 and 5 mm and said low relief zones have circular form or any other suitable form, e.g. rectangular or square form.

13. Device according to any one of the claims 1 to 12, wherein said low relief zones form concave cavities, with depth comprised between 0.1 and 5 mm.

14. Device according to any one of the claims 1 to 13, wherein said device comprises, on the internal face, a double series of low relief zones (25a, 25b, 25c, 25d, 25e, 25f) which correspond with two adhesive portions (27a), opposite each other and externally situated with respect to the two non-adhesive portions (27b) comprising the low relief zones on which they will be folded.

15. Device according to any one of the claims 1 to 14, which comprises a protective housing portion (40) and a tongue portion (31) which is extended from one edge of the protective housing portion (40), wherein said protective housing portion (40) is extended along a main axis so as to define two opposite greater edges (40') and two opposite smaller edges (40") and is divided by a line of demarcation (41) perpendicular to said greater edges (40') which divides said protective housing portion (40) into two non-symmetrical portions, a smaller portion (40a) and a greater portion (40b), wherein said greater portion (40b) comprises an end portion (42), in turn separated by a line of demarcation, which will serve as a closure of said protective housing portion, wherein said tongue portion (31), integrally made with the protective housing portion (40), is extended from a greater edge (40') of said smaller portion (40a) of said protective housing portion (40), perpendicular to the main axis of the protective housing portion (40) and wherein said tongue portion (31) comprises, adjacent to the edge of the protective housing portion (40) from which it extends, an adhesive portion (33) which is covered by an adhesive film and wherein the distal end of said tongue portion (31) constitutes gripping means (36).

16. Device according to any one of the claims 1 to 15, wherein said protective housing portion (40) is folded to form the protective housing and the tongue portion (31) is inserted in the protective housing itself, so as to make the distal end (36) of the tongue portion (31) come out from the edge of the protective housing opposite the edge from which the tongue portion (31) is extended and to make said adhesive portion (33) project from the edge of the protective housing itself.

17. Device according to any one of the claims 1 to 16, wherein it is possible to detach the adhesive film (2, 21, 33) from the adhesive portion (7a, 27a, 33), by using a bi-adhesive film selected in such a manner that it has greater adhesive strength on the side in contact with the support than the adhesive strength present on the side to be used for the sample collection.

18. Device according to any one of the claims 1 to 17, wherein said adhesive film (2, 21, 33) is constituted by one or more separatable portions, in particular it can comprise 1 to 10 adhesive strips, preferably 6 adhesive strips, arranged in a contiguous manner on the support in order to form the total adhesive surface.

19. Device according to any one of the claims 1 to 18, previously treated with DiEthylPyroCarbonate.

20. Device according to any one of the claims 1 to 19, previously sterilized, preferably in an autoclave.

21. Device according to any one of the claims 1 to 20, wherein the device is part of a kit which also comprises a bag, preferably impermeable and sealable, where the device itself is inserted and housed.

22. Device according to any one of the claims 1 to 21, which comprises detachable self-adhesive labels containing an ID code of the sample, constituted by an alphanumeric code or a bar code, wherein said labels can be present on said sheet (7, 27) on said protective housing portion (40) or on said tongue portion (31), or on a portion added for such purpose and positioned in such a manner so as to not interfere with the use of the device itself.

23. Use of the device according to any one of the claims 1 to 22 for the collection of skin cells and the subsequent extraction of DNA and/or RNA from the cells themselves.
